# EUROPÄISCHE PATENTSCHRIFT

(11) **EP 0 888 288 B1**
(45) Veröffentlichungstag und Bekanntmachung des Hinweises auf die Patenterteilung: **13.06.2001**
(21) Anmeldenummer: 97906186.8
(22) Anmeldetag: 07.03.1997
(51) Int. Cl.: C07C 233/58, C07C 233/59, C07C 233/60, C07C 255/47, C07C 255/60, A01N 37/18

(54) **CARBONSÄUREAMIDE MIT FUNGIZIDER WIRKUNG**
FUNGICIDALLY ACTIVE CARBOXYLIC ACID AMIDES
AMIDES D'ACIDE CARBOXYLIQUE A ACTION FONGICIDE

(30) Priorität: 22.03.1996 DE 19611350
(43) Veröffentlichungstag der Anmeldung: 07.01.1999
(73) Patentinhaber: BASF AKTIENGESELLSCHAFT, 67056 Ludwigshafen (DE)
(72) Erfinder: WETTERICH, Frank, D-67112 Mutterstadt (DE); WAGNER, Oliver, D-66450 Bexbach (DE); EICKEN, Karl, D-67157 Wachenheim (DE); AMMERMANN, Eberhard, D-64646 Heppenheim (DE); STRATHMANN, Siegfried, D-67117 Limburgerhof (DE); LORENZ, Gisela, D-67434 Hambach (DE); SPEAKMAN, John-Bryan, D-67273 Bobenheim (DE)
(86) Internationale Anmeldenummer: EP9701161
(87) Internationale Veröffentlichungsnummer: WO9735838

(56) Entgegenhaltungen:
- EP-A- 0 653 418
- DE-A- 2 519 943
- DE-A- 2 705 601
- CHEMICAL AND PHARMACEUTICAL BULLETIN, Bd. 28, Nr. 2, Februar 1980, TOKYO JP, Seiten 453-458, XP002031763 YUKIO FUJITA ET AL.: "New Hypocholesterolemic Abietamide Derivatives. I. Struture-activity Relationship"
- JOURNAL OF ORGANIC CHEMISTRY, Bd. 43, Nr. 11, 26.Mai 1978, EASTON US, Seiten 2232-2236, XP002031764 H. NUMAN ET AL. : "Deuterium and the Octant rule for Ketones. Syntheses and Circular Dichroism Data of Chiral 4-Deuterioadamantan-2-ones"
- JOURNAL OF ORGANIC CHEMISTRY, Bd. 58, Nr. 11, 21.Mai 1993, EASTON US, Seiten 3148-3155, XP002031765 C. HAMDOUCHI ET AL.: "Surface Nature of Grignard Reagent Formation. Chiral 1-Methylspiro[2.5]octylmagnesium Bromide"
- CHEMICAL ABSTRACTS, vol. 66, no. 25, 19.Juni 1967 Columbus, Ohio, US; abstract no. 115356a, F. N. STEPANOV ET AL.: "Adamantane and its derivatives. XI. Molecular asymmetry of adamantane derivatives" Seite 10707; Spalte 1; XP002031767 & ZH. ORGAN. KHIM., Bd. 2, Nr. 9, 1966, Seiten 1635-1638,
- JOURNAL OF ORGANIC CHEMISTRY, Bd. 24, Nr. 12, 18.Februar 1960, EASTON US, Seiten 1907-1914, XP002031766 CARLETON W. ROBERTS ET AL.: "Organic Chemistry of alpha-Methylstyrene. I. Reactions Leading to N-(alpha,alpha- dimethyl-substituted-benzyl)acrylamides"
- DATABASE CROSSFIRE Beilstein Informationssysteme GmbH, Frankfurt DE XP002031768 & JP 04 955 758 A (MURAI KHIROSI ET AL.) 1977
- DATABASE CROSSFIRE Beilstein Informationssysteme GmbH, Frankfurt DE XP002031769 & ANAL. CHEM., Bd. 45, 1973, Seiten 896-900,
- DATABASE CROSSFIRE Beilstein Informationssysteme GmbH, Frankfurt DE XP002031770 & SYNTH. COMMUN., Bd. 19, 1989, Seiten 993-1000,
- DATABASE CROSSFIRE Beilstein Informationssysteme GmbH, Frankfurt DE XP002031771 & INDIAN J. CHEM. SEC. B, Bd. 26, 1987, Seiten 1-12,
- DATABASE CROSSFIRE Beilstein Informationssysteme GmbH, Frankfurt DE XP002031772 & J. ORG. CHEM. USSR , Bd. 7, 1971, Seite 2221
- DATABASE CROSSFIRE Beilstein Informationssysteme GmbH, Frankfurt DE XP002031773 & TETRAHEDRON, Bd. 42, 1986, Seiten 4035-4044,
- DATABASE CROSSFIRE Beilstein Informationssysteme GmbH, Frankfurt DE XP002031774 & SOV. PROG. CHEM., Bd. 38, 1972, Seite 74
- DATABASE WPI Section Ch, Week 9601 Derwent Publications Ltd., London, GB; Class C03, AN 96-010844 XP002031775 in der Anmeldung erwähnt & WO 95 31432 A (SUMITOMO CHEM.) 23.November 1995

## Beschreibung

Die vorliegende Erfindung betrifft Carbonsäureamide der Formel I 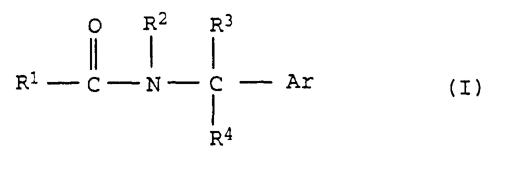 in der die Substituenten die folgenden Bedeutungen haben:
- R¹: C₆-C₁₅-Bicycloalkyl, Tricyclo[3.3.1.1.^{3,7}]decan, Tricyclo[5.2.1.0^{2,6}] decan oder C₇-C₁₅-Bicycloalkenyl, wobei diese Reste partiell oder vollständig halogeniert sein können und/oder, sofern sie nicht vollständig halogeniert sind, eine oder unabhängig voneinander zwei, drei, vier oder fünf der folgenden Gruppen tragen können: Cyano, C₁-C₄-Alkyl, C₁-C₄-Halogenalkyl, C₁-C₄-Alkoxy, C₁-C₄-Halogenalkoxy und Aryl, wobei das Aryl partiell oder vollständig halogeniert sein und/oder einen oder unabhängig voneinander zwei oder drei der folgenden Substituenten tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio;
- R²: Wasserstoff,
- R³: Wasserstoff,
- Ar: Aryl oder Heteroaryl, wobei diese Reste eine oder unabhängig voneinander zwei oder drei der folgenden Gruppen tragen können: Halogen, Cyano, C₁-C₄-Alkyl, C₁-C₄-Alkoxyalkyl, C₁-C₄-Halogen-, alkyl, C₁-C₄-Alkoxy, C₁-C₄-Halogenalkoxy, C₁-C₄-Alkylthio, C₁-C₄-Alkoxycarbonyl, Aryl, Aryloxy und Heteroaryl, wobei in diesen Gruppen die Ringe ihrerseits einen oder unabhängig voneinander zwei oder drei der folgenden Substituenten tragen können: Halogen, Cyano, C₁-C₄-Alkyl, C₁-C₄-Alkoxyalkyl, C₁-C₄-Halogen-alkyl, C₁-C₄-Alkoxy, C₁-C₄-Halogenalkoxy, C₁-C₄-Alkylthio und C₁-C₄-Alkoxycarbonyl,
- R⁴: C₁-C₈-Alkyl, welches partiell oder vollständig halogeniert sein und/oder eine oder zwei der folgenden Gruppen tragen kann: C₁-C₄-Alkoxy, C₁-C₄-Halogenalkoxy, C₁-C₄-Alkylthio, C₃-C₇-Cycloalkyl, C₅-C₇-Cycloalkenyl, wobei die cyclischen Gruppen ihrerseits ein oder unabhängig voneinander zwei oder drei Halogenatome, C₁-C₃-Alkylgruppen und/oder C₁-C₃-Alkoxygruppen tragen können und Aryl, wobei das Aryl partiell oder vollständig halogeniert sein und/oder einen oder unabhängig voneinander zwei oder drei der folgenden Substituenten tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio oder
C₃-C₆-Cycloalkyl, C₃-C₆-Cycloalkenyl oder Heterocyclyl, wobei diese Reste partiell oder vollständig halogeniert sein und/oder eine oder unabhängig voneinander zwei oder drei der folgenden Gruppen tragen können: Cyano, C₁-C₆-Alkyl, C₁-C₆-Halogenalkyl, C₂-C₆-Alkenyl, C₁-C₆-Alkoxy und C₁-C₆-Alkylthio;
mit der Maßgabe, daß Ar nicht unsubstituiertes Phenyl ist, wenn R¹ Tricyclo[3.3.1.1^{3,7}]decan bedeutet,
ausgenommen 2-Cyano-N-[1-(1-naphthyl)ethyl]-3-phenylbicyclo[2.2.1]hept-5-en-2-carboxamid, Endo-bicyclo[3.3.1]non-6-en-3-carboxy-(1-phenyl)ethylamid, (5R,6S)-6-Methylbicyclo[2.2.1]hept-2-en-5-carboxy-R-1-(1-naphthyl)ethylamid, 2,5,5,8a-Tetramethyldecahydronaphthalin-l-carbonsäure(l-phenyl)ethylamid.

Außerdem betrifft die Erfindung ein Verfahren zur Herstellung der Verbindungen I, die Verbindungen I enthaltende Mittel und die Verwendung der Verbindungen I zur Herstellung der Mittel.

Weiterhin betrifft die Erfindung ein Verfahren zur Bekämpfung von Schadpilzen und die Verwendung der Verbindungen I hierzu.

Aus JP-A 02/233 654, EP-A 170 842 und WO-A 95/31432 sind fungizide Benzylamide bekannt. Fungizide Amide bicyclischer Carbonsäuren sind bekannt aus EP-A 653 418.

In einer Arbeit über asymmetrische Diels-Alder Reaktionen werden ferner einige Isomere von 2-Cyano-N-[1-(1-naphthyl)ethyl]-3-phenylbicyclo[2.2.1]hept-5-en-2-carboxamid genannt (J. Org. Chem. 57, (1992) Seite 4664 bis Seite 4669).

Die Verbindungen aus JP-A 02/233 654, EP 170 842 und WO-A 95/31456 vermögen jedoch hinsichtlich ihrer fungiziden Wirkung noch nicht zu befriedigen.

Der vorliegenden Erfindung lagen daher neue Carbonsäureamide mit verbesserter Wirkung gegen Schadpilze als Aufgabe zugrunde.

Demgemäß wurden die eingangs definierten Verbindungen I gefunden.

Außerdem wurde ein Verfahren zur Herstellung der Verbindungen I, die Verbindungen I enthaltende Mittel und die Verwendung der Verbindungen I zur Herstellung der Mittel gefunden sowie ein Verfahren zur Bekämpfung von Schadpilzen und die Verwendung der Verbindungen I hierzu.

Die Verbindungen I können ausgehend von den entsprechenden Carbonsäuren IIIa

R¹-COOH (IIIa)

durch Umsetzung mit Aminen II hergestellt werden (die Literaturzitate "Houben-Weyl" beziehen sich auf: Houben-Weyl, Methoden der Organischen Chemie, 4. Auflage, Thieme Verlag, Stuttgart).

Die Carbonsäuren IIIa sind bekannt aus der EP-A 653 418.

Die Amine II sind ebenfalls allgemein bekannt oder nach bekannten Methoden erhältlich (vgl. WO-A 95/23 784).

Vorzugsweise arbeitet man so, daß man zunächst die Carbonsäuren IIIa in carboxyaktivierte Derivate III, vor allem in Acylhalogenide - z.B. die Chloride -, Acylcyanide oder Anhydride, überführt (vgl. Tetrahedron Letters, Band 18, Seite 1595 bis Seite 1598 (1973) bzw. "Houben-Weyl", Band 15/1, Seite 28 bis Seite 32). Diese Derivate III werden dann mit den Aminen II in Gegenwart von Basen zur Reaktion gebracht.

Zur Herstellung der Acylcyanide eignet sich z.B. die Reaktion der Carbonsäuren IIIa mit Cyanphosphonsäurediethylester, vor allem in einem inerten Lösungsmittel wie Tetrahydrofuran, Toluol oder Dichlormethan.

Zur Herstellung der carboxyaktivierten Anhydride ist die Umsetzung der Carbonsäuren IIIa mit Kohlensäurechloriden wie Chlorameisensäure-iso-butylester in Gegenwart von Basen und gegegebenfalls in einem inerten Lösungsmittel wie Toluol oder Tetrahydrofuran bevorzugt.

Die Umsetzung der Amine II mit den carboxyaktivierten Carbonsauren III erfolgt vorzugsweise in einem Lösungsmittel wie Dichlormethan, Tetrahydrofuran oder Toluol.

Als Basen können insbesondere die Amine II selbst dienen, wobei man sie aus dem Rohprodukt üblicherweise zurückgewinnt.

In einer bevorzugten Ausführungsform dieser Verfahrensstufe weri den die Carbonsäure IIIa, das Amin II, das zur Erzeugung des carboxyaktivierten Derivates der Carbonsäure III geeignete Reagenz und die Base im Eintopfverfahren, gegebenenfalls in einem inerten Lösungsmittel, zur Reaktion gebracht.

Das derart erhaltene Reaktionsgemisch wird in üblicher Weise auf die Verbindungen I aufgearbeitet, z.B. durch Mischen mit Wasser, Trennung der Phasen und gegebenenfalls chromatographische Reinigung der Rohprodukte. Die Endprodukte fallen z.T. in Form farbloser oder schwach bräunlicher, zäher Öle an, die unter verminderi tem Druck und bei mäßig erhöhter Temperatur von flüchtigen Anteilen befreit werden können. Sofern die Endprodukte als Feststoffe erhalten werden, kann die Reinigung auch beispielsweise durch Umkristallisieren oder Digerieren erfolgen.

Die Verbindungen der Formel I können gegebenenfalls in Abhängigkeit von der Art der Substituenten als geometrische und/oder optische Isomere oder Isomerengemische vorliegen. Insbesondere kann in den Verbindungen I das Kohlenstoffatom, welches die Gruppen R³ und R⁴ trägt, R- oder S-Konfiguration gemäß der IUPAC-Nomenklatur aufweisen. Sowohl die reinen hier beschriebenen Isomeren als auch die Gemische der Isomeren haben fungizide Wirkung.

In den Verbindungen I kann bezüglich des Restes R¹ der übrige Teil des Moleküls exo oder endo angeordnet sein. Beide Isomeren sowie deren Gemische sind jeweils fugizid wirksam.

Bei der eingangs angegebenen Definition der Verbindungen I wurden Sammelbegriffe verwendet, die allgemein repräsentativ für die folgenden Substituenten stehen:

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome wie vorstehend genannt ersetzt sein können.
Bicycloalkyl: bicyclische Alkylgruppen mit 6 bis 15 Kohlenstoffringgliedern, z.B. Bicyclo-[2.1.1]-hex-5-yl, Bicyclo-[2.2.1]hept-2-yl, Bicyclo-[2.2.2]oct-2-yl, Bicyclo-[3.2.1]-oct-6-yl, Bicyclo-[3.2.2]-non-6-yl, Bicyclo-[4.2.2]-dec-7-yl, Bicyclo-[3.1.0]-hex-1-yl, Bicyclo-[4.1.0]-hept-1-yl, Bicyclo-[4.3.0]-non-1-yl, Bicyclo-[4.4.0]-dec-1-yl, besonders bevorzugt 5-Methyl-bicyclo-[2.1.1]-hex-5-yl, 2-Methyl-bicyclo-[2.2.1]hept-2-yl, 2-Methyl-bicyclo-[2.2.2]oct-2-yl, 6-Methyl-bicyclo-[3.2.1]-oct-6-yl, 6-Methyl-bicyclo-[3.2.2]-non-6-yl, 7-Methyl-bicyclo-[4.2.2]-dec-7-yl, 1-Methyl-bicyclo-[3.1.0]-hex-1-yl, 1-Methyl-bicyclo-[4.1.0]-hept-1-yl, 1-Methyl-bicyclo-[4.3.0]-non-1-yl, 1-Methyl-bicyclo-[4.4.0]-dec-1-yl, 2-Methyl-bicyclo-[3.1.0]-hex-1-yl, 2-Methyl-bicyclo-[4.1.0]-hept-1-yl, 2-Methyl-bicyclo-[4.3.0]-non-1-yl, 2-Methyl-bicyclo-[4.4.0]-dec-1-yl;
Tricycloalkyl: Tricyclo[3.3.1.1^{3.7}]decan (Adamantyl), Tricyclo [5.2.1.0^{2.6}] decan;
Bicycloalkenyl: bicyclische Alkenylgruppen mit 7 bis 15 Kohlenstoffringgliedern, z.B. Bicyclo-[2.2.1]-hept-2-en-5-yl, Bicyclo-[2.2.2]-oct-2-en-5-yl, Bicyclo-[4.2.2]-dec-7-en-2-yl, Bicyclo-[4.3.0]-non-7-en-1-yl, Bicyclo-[4.4.0]-dec-3-en-1-yl, Bicyclo-[4.1.0]-hept-3-en-1-yl, 5-Methyl-bicyclo-[2.2.1]hept-2-en-5-yl, 5-Methyl-bicyclo-[2.2.2]-oct-2-en-5-yl, 2-Methylbicyclo-[4.2.2]-dec-7-en-2-yl, 2-Methyl-bicyclo-[4.3.0]non-7-en-1-yl, 2-Methyl-bicyclo-[4.4.0]-dec-3-en-1-yl, 2-Methylbicyclo-[4.1.0]-hept-3-en-1-yl;
Halogen: Fluor, Chlor, Brom und Jod;
Alkyl: geradkettige oder verzweigte Alkylgruppen mit 1 bis 8 Kohlenstoffatomen, z.B. C₁-C₆-Alkyl wie Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 2,2-Dimethylpropyl, 1,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 2,2-Dimethylbutyl, 3,3-Dimethylbutyl, 1,2-Dimethylbutyl, 1,3-Dimethylbutyl, 2,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Alkyliden: geradkettige oder verzweigte Alkylidengruppen mit 3 bis 5 Kohlenstoffatomen, z.B. 1,3-Propyliden, 1,4-Butyliden, 1-Methyl-1,3-propyliden, 2-Methyl-1,3-propyliden, 2,2-Dimethyl-1,3-propyliden, 1,5-Pentyliden, 1-Methyl-1,4-butyliden;
Halogenalkyl bzw. partiell oder vollständig halogeniertes Alkyl: geradkettige oder verzweigte Alkylgruppen mit 1 bis 4 bzw. 8 Kohlenstoffatomen (wie vorstehend genannt), wobei in diesen Gruppen die Wasserstoffatome partiell oder vollständig durch Halogenatome (wie vorstehend genannt) ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkoxygruppen mit 1 bis 4 Kohlenstoffatomen, z.B. C₁-C₃-Alkoxy wie Methyloxy, Ethyloxy, Propyloxy und 1-Methylethyloxy;
Alkoxyalkyl: geradkettige oder verzweigte Alkylgruppen mit 1 bis 8 Kohlenstoffatomen (wie vorstehend genannt), welche in einer beliebigen Position eine geradkettige oder verzweigte Alkoxygruppe (wie vorstehend genannt) mit im Falle von C₁-C₄-Alkoxyalkyl 1 bis 4 Kohlenstoffatomen tragen, wie Methoxymethyl, Ethoxymethyl, n-Propoxymethyl, n-Butoxymethyl, 1-Methoxyethyl, 2-Methoxyethyl, 1-Ethoxyethyl, 2-Ethoxyethyl, 2-n-Propoxyethyl und 2-Butoxyethyl;
Halogenalkoxy: geradkettige oder verzweigte Alkoxygruppen mit 1 bis 4 Kohlenstoffatomen (wie vorstehend genannt), wobei in diesen Gruppen die Wasserstoffatome partiell oder vollständig durch Halogenatome (wie vorstehend genannt) ersetzt sein können, z.B. C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
Alkylthio: geradkettige oder verzweigte Alkylgruppen mit 1 bis 4 Kohlenstoffatomen (wie vorstehend genannt), welche über ein Schwefelatom (-S-) an das Gerüst gebunden sind, z.B. C₁-C₄-Alkylthio wie Methylthio, Ethylthio, Propylthio, 1-Methylethylthio, n-Butylthio und tert.-Butylthio;
Alkoxycarbonyl: geradkettige oder verzweigte Alkoxygruppen mit 1 bis 4 C-Atomen (wie vorstehend genannt), welche über eine Carbonylgruppe (-CO-) an das Gerüst gebunden sind;
Alkenyl: geradkettige oder verzweigte Alkenylgruppen mit 2 bis 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 2-Methyl-1-propenyl, 1-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und l-Ethyl-2-methyl-2-propenyl;
Alkinyl: geradkettige oder verzweigte Alkinylgruppen mit 2 bis 8 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-l-butinyl, 1-Ethyl-2-butinyl, 1-Ethyl-3-butinyl, 2-Ethyl-3-butinyl und 1-Ethyl-1-methyl-2-propinyl;
Cycloalkyl: monocyclische Alkylgruppen mit 3 bis 7 Kohlenstoffringgliedern, z.B. C₃-C₇-Cycloalkyl wie Cyclopropyl, Cyclobutyl, Cyclopentyl, Cyclohexyl und Cycloheptyl;
Cycloalkenyl: monocyclische Alkylgruppen mit 5 bis 7 Kohlenstoffringgliedern die eine oder mehrere Doppelbindungen enthalten z.B. C₅-C₇-Cycloalkenyl wie Cyclopentenyl, Cyclohexenyl und Cycloheptenyl;
Heterocyclyl: drei- bis sechsgliedrige, gesättigte oder partiell ungesättigte mono- oder polycyclische Heterocyclen, die ein bis drei Heteroatome ausgewählt aus einer Gruppe bestehend aus Sauerstoff, Stickstoff und Schwefel enthalten, und welche an das Gerüst gebunden sind, z.B. 2-Tetrahydrofuranyl, Oxiranyl, 3-Tetrahydrofuranyl, 2-Tetrahydrothienyl, 3-Tetrahydrothienyl, 2-Pyrrolidinyl, 3-Pyrrolidinyl, 3-Isoxazoldinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,3-Dihydro-fur-4-yl, 2,3-Dihydro-fur-5-yl, 2,5-Dihydro-fur-2-yl, 2,5-Dihydro-fur-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisothiazol-3-yl, 2,5-Dihydroisothiazol-4-yl, 2,5-Dihydroisothiazol-5-yl, 2,3-Dihydroisopyrazol-3-yl, 2,3-Dihydroisopyrazol-4-yl, 2,3-Dihydroisopyrazol-5-yl, 4,5-Dihydroisopyrazol-3-yl, 4,5-Dihydroisopyrazol-4-yl, 4,5-Dihydroisopyrazol-5-yl, 2,5-Dihydroisopyrazol-3-yl, 2,5-Dihydroisopyrazol-4-yl, 2,5-Dihydroisopyrazol-5-yl, 2,3-Dihydrooxazol-3-yl, 2,3-Dihydrooxazol-4-yl, 2,3-Dihydrooxazol-5-yl, 4,5-Dihydrooxazol-3-yl, 4,5-Dihydrooxazol-4-yl, 4, 5-Dihydrooxazol-5-yl, 2,5-Dihydrooxazol-3-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 1,3-Dioxolan-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl, 1,3-Dithian-2-yl,
Aryl: monocyclische oder polycyclische aromatische Gruppen mit 6 bis 10 C-Atomen wie Phenyl und Naphthyl;
Arylalkyl: Arylgruppen (wie vorstehend genannt), welche im Falle von Aryl-(C₁-C₄)-alkyl über Alkylgruppen mit 1 bis 4 Kohlenstoffatomen (wie vorstehend genannt) an das Gerüst gebunden sind, z.B. Phenyl-(C₁-C₄)-alkyl wie Benzyl, 2-Phenylethyl, 3-Phenylpropyl, 4-Phenylbutyl, 1-Phenylethyl, 1-Phenylpropyl und 1-Phenylbutyl;
Aryloxy: Arylgruppen (wie vorstehend genannt), welche über ein Sauerstoffatom (-O-) an das Gerüst gebunden sind wie Phenoxy, 1-Naphthoxy und 2-Naphthoxy;

Heteroaryl: aromatische mono- oder polycyclische Reste, welche neben Kohlenstoffringgliedern zusätzlich 1 bis 4 Stickstoffatome oder 1 bis 3 Stickstoffatome und ein Sauerstoff- oder ein Schwefelatom oder ein Sauerstoff- oder ein Schwefelatom enthalten können, z.B.:
- 5-gliedriges Heteroaryl, enthaltend 1 bis 3 Stickstoffatome: 5-Ring-Heteroarylgruppen, welche neben Kohlenstoffatomen 1 bis 3 Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4-Triazol-3-yl und 1,3,4-Triazol-2-yl;
- 5-gliedriges Heteroaryl, enthaltend 1 bis 4 Stickstoffatome oder 1 bis 3 Stickstoffatome und 1 Schwefelatom oder Sauerstoffatom oder 1 Sauerstoff- oder 1 Schwefelatom: 5-RingHeteroarylgruppen, welche neben Kohlenstoffatomen 1 bis 4 Stickstoffatome oder 1 bis 3 Stickstoffatome und 1 Schwefel- oder Sauerstoffatom oder 1 Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl;
- benzokondensiertes 5-gliedriges Heteroaryl, enthaltend 1 bis 3 Stickstoffatome oder 1 Stickstoffatom und/oder ein Sauerstoff- oder Schwefelatom: 5-Ring-Heteroarylgruppen, welche neben Kohlenstoffatomen 1 bis 4 Stickstoffatome oder 1 bis 3 Stickstoffatome und 1 Schwefel- oder Sauerstoffatom oder 1 Sauerstoff- oder ein Schwefelatom als Ringglieder enthalten können, und in welchen 2 benachbarte Kohlenstoffringglieder oder 1 Stickstoff- und 1 benachbartes Kohlenstoffringglied durch eine Buta-1,3-dien-1,4-diylgruppe verbrückt sein können;
- über Stickstoff gebundenes 5-gliedriges Heteroaryl, enthaltend 1 bis 4 Stickstoffatome, oder über Stickstoff gebundenes benzokondensiertes 5-gliedriges Heteroaryl, enthaltend 1 bis 3 Stickstoffatome: 5-Ring-Heteroarylgruppen, welche neben Kohlenstoffatomen 1 bis 4 Stickstoffatome bzw. 1 bis 3 Stickstoffatome als Ringglieder enthalten können, und in welchen 2 benachbarte Kohlenstoffringglieder oder ein Stickstoff- und ein benachbartes Kohlenstoffringglied durch eine Buta-1,3-dien-1,4-diylgruppe verbrückt sein können, wobei diese Ringe über eines der Stickstoffringglieder an das Gerüst gebunden sind;
- 6-gliedriges Heteroaryl, enthaltend 1 bis 3 bzw. 1 bis 4 Stickstoffatome: 6-Ring-Heteroarylgruppen, welche neben Kohlenstoffatomen 1 bis 3 bzw. 1 bis 4 Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
- benzokondensiertes 6-gliedriges Heteroaryl, enthaltend 1 bis 4 Stickstoffatome: 6-Ring-Heteroarylgruppen, in welchen 2 benachbarte Kohlenstoffringglieder durch eine Buta-1,3-dien-1,4-diylgruppe verbrückt sein können, z.B. Chinolin, Isochinolin, Chinazolin und Chinoxalin.

Im Hinblick auf ihre biologische Wirkung gegen Schadpilze sind Verbindungen I bevorzugt, in denen das Kohlenstoffatom, welches die Gruppen R³ und R⁴ trägt, R-Konfiguration hat.

Außerdem sind Verbindungen I bevorzugt, in denen R³ für Wasserstoff und R⁴ für C₁-C₄-Alkyl, vor allem Methyl, steht.

Weiterhin sind Verbindungen I bevorzugt, in denen Ar für gegebenenfalls substituiertes Phenyl steht, welches insbesondere in der 2-Position oder in der 2- und 4-Position und vor allem in der 4-Position substituiert ist. Bevorzugte alleinige Substituenten in der 4-Position sind Cyano, vorzugsweise Methyl und insbesondere Halogen, vor allem Chlor.

Darüberhinaus sind Verbindungen I bevorzugt, in denen R¹ für gegebenenfalls substituiertes Bicycloalkyl mit 6 bis 10 Kohlenstoffatomen steht.

Außerdem sind Verbindungen I bevorzugt, in denen R¹ für gegebenenfalls substituiertes Bicycloalkenyl mit 7 bis 10 Kohlenstoffatomen steht.

Insbesondere sind im Hinblick auf ihre Verwendung die in den anschließenden Tabellen zusammengestellten Verbindungen I bevorzugt.

### Tabelle 1:

Verbindungen der allgemeinen Formel I.1, in welcher die Bedeutungen der Kombinationen aus Z^{1,} Z² und "*" durch die Zeilen der Tabelle A gegeben sind

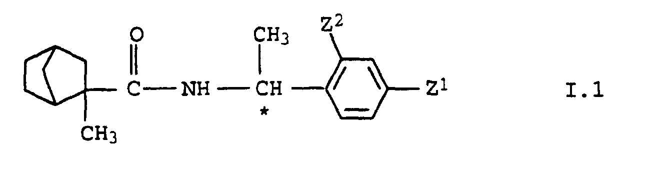

### Tabelle 2:

Verbindungen der allgemeinen Formel I.2, in welcher die Bedeutungen der Kombinationen aus Z¹, Z² und "*" durch die Zeilen der Tabelle A gegeben sind

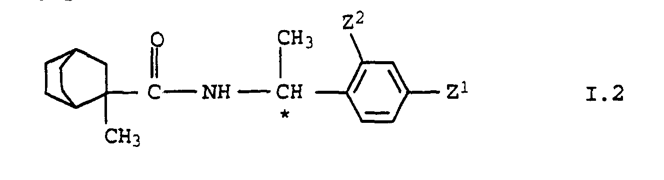

### Tabelle 3:

Verbindungen der allgemeinen Formel I.3, in welcher die Bedeutungen der Kombinationen aus Z^{1,} Z² und "*" durch die Zeilen der Tabelle A gegeben sind

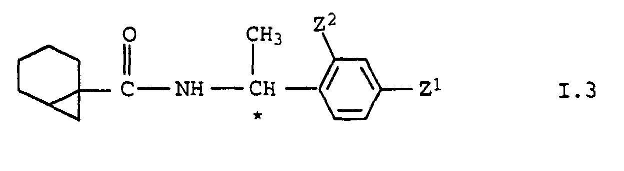

### Tabelle 4:

Verbindungen der allgemeinen Formel I.4, in welcher die Bedeutungen der Kombinationen aus Z^{1,} Z² und "*" durch die Zeilen der Tabelle A gegeben sind

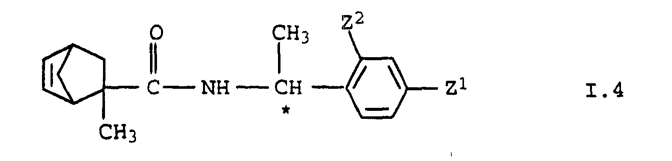

### Tabelle 5:

Verbindungen der allgemeinen Formel I.5, in welcher die Bedeutungen der Kombinationen aus Z¹, Z² und "*" durch die Zeilen der Tabelle A gegeben sind

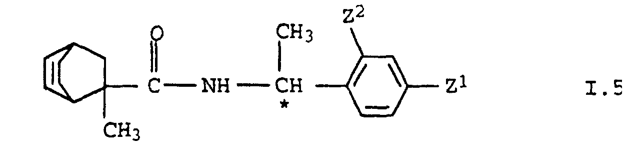

### Tabelle 6:

Verbindungen der allgemeinen Formel I.6, in welcher die Bedeutungen der Kombinationen aus Z¹, Z² und "*" durch die Zeilen der Tabelle A gegeben sind

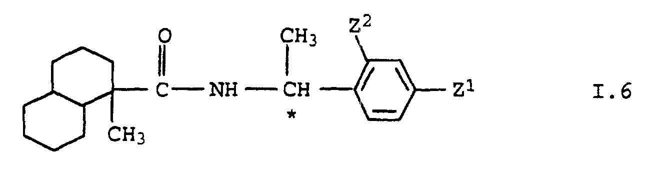

### Tabelle 7:

Verbindungen der allgemeinen Formel I.7, in welcher die Bedeutungen der Kombinationen aus Z^{1,} Z² und "*" durch die Zeilen der Tabelle A gegeben sind

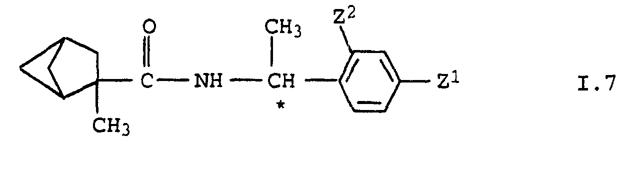

### Tabelle 8:

Verbindungen der allgemeinen Formel I.8, in welcher die Bedeutungen der Kombinationen aus Z¹, Z² und "*" durch die Zeilen der Tabelle A gegeben sind

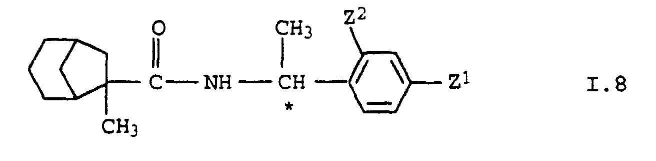

### Tabelle 9:

Verbindungen der allgemeinen Formel I.9, in welcher die Bedeutungen der Kombinationen aus Z¹' Z² und "*" durch die Zeilen der Tabelle A gegeben sind

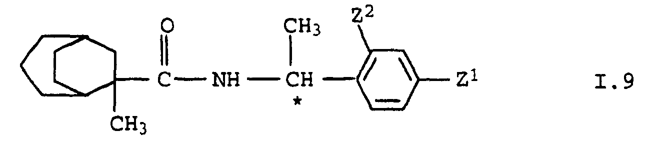

### Tabelle 10:

Verbindungen der allgemeinen Formel I.10, in welcher die Bedeutungen der Kombinationen aus Z^{1,} Z² und "*" durch die Zeilen der Tabelle A gegeben sind

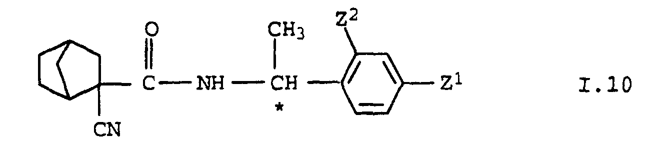

### Tabelle 11:

Verbindungen der allgemeinen Formel I.11, in welcher die Bedeutungen der Kombinationen aus Z^{1,} Z² und "*" durch die Zeilen der Tabelle A gegeben sind

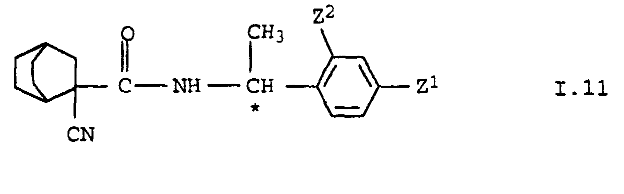

### Tabelle 12:

Verbindungen der allgemeinen Formel I.12, in welcher die Bedeutungen der Kombinationen aus Z¹, Z² und "*" durch die Zeilen der Tabelle A gegeben sind

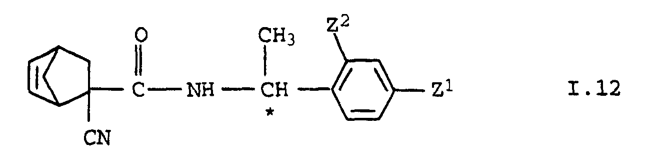

### Tabelle 13:

Verbindungen der allgemeinen Formel I.13, in welcher die Bedeutungen der Kombinationen aus Z^{1,} Z² und "*'' durch die Zeilen der Tabelle A gegeben sind

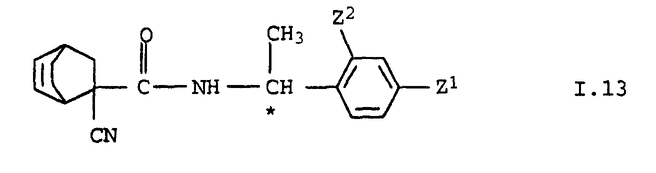

### Tabelle 14:

Verbindungen der allgemeinen Formel I.14, in welcher die Bedeutungen der Kombinationen aus Z^{1,} Z² und "*" durch die Zeilen der Tabelle A gegeben sind

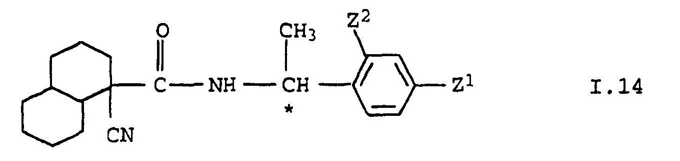

### Tabelle 15:

Verbindungen der allgemeinen Formel I.15, in welcher die Bedeutungen der Kombinationen aus Z^{1,} Z² und "*" durch die Zeilen der Tabelle A gegeben sind

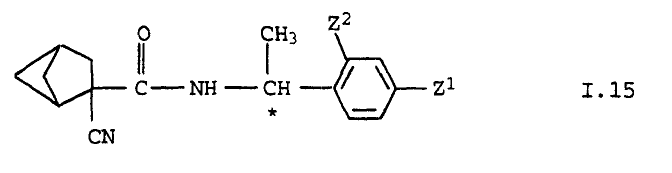

### Tabelle 16:

Verbindungen der allgemeinen Formel I.16, in welcher die Bedeutungen der Kombinationen aus Z^{1,} Z² und "*" durch die Zeilen der Tabelle A gegeben sind

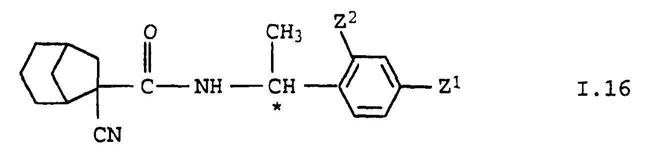

### Tabelle 17:

Verbindungen der allgemeinen Formel I.17, in welcher die Bedeutungen der Kombinationen aus Z^{1,} Z² und "*" durch die Zeilen der Tabelle A gegeben sind

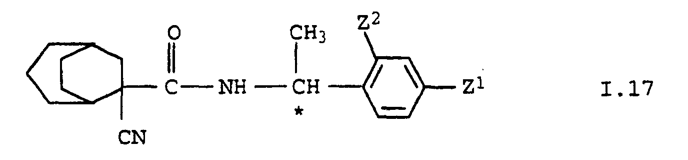

### Tabelle 18:

Verbindungen der allgemeinen Formel I.18, in welcher die Bedeutungen der Kombinationen aus Z^{1,} Z² und "*" durch die Zeilen der Tabelle A gegeben sind

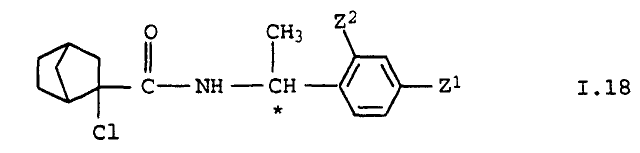

### Tabelle 19:

Verbindungen der allgemeinen Formel I.19, in welcher die Bedeutungen der Kombinationen aus Z^{1,} Z² und "*" durch die Zeilen der Tabelle A gegeben sind

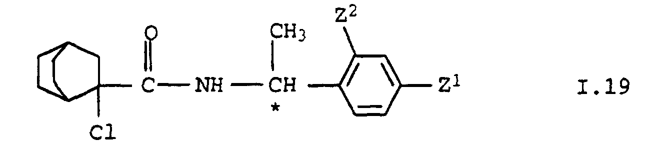

### Tabelle 20:

Verbindungen der allgemeinen Formel I.20, in welcher die Bedeutungen der Kombinationen aus z¹, Z² und "*" durch die Zeilen der Tabelle A gegeben sind

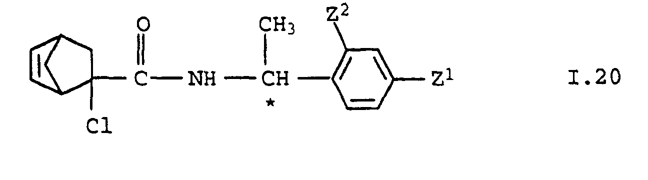

### Tabelle 21:

Verbindungen der allgemeinen Formel I.21, in welcher die Bedeutungen der Kombinationen aus Z^{1,} Z² und "*" durch die Zeilen der Tabelle A gegeben sind

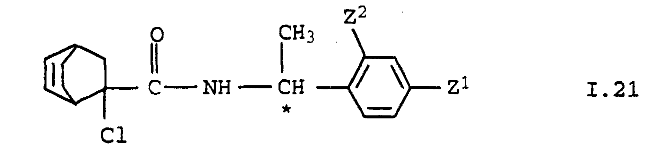

### Tabelle 22:

Verbindungen der allgemeinen Formel I.22, in welcher die Bedeutungen der Kombinationen aus Z^{1,} Z² und "*" durch die Zeilen der Tabelle A gegeben sind

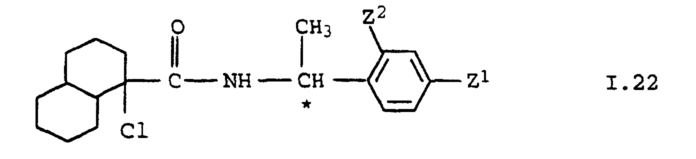

### Tabelle 23:

Verbindungen der allgemeinen Formel I.23, in welcher die Bedeutungen der Kombinationen aus Z^{1,} Z² und "*" durch die Zeilen der Tabelle A gegeben sind

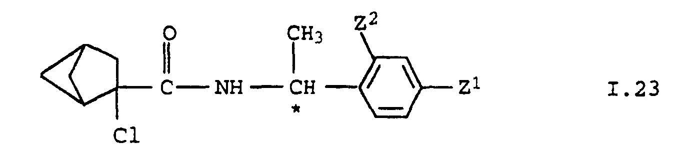

### Tabelle 24:

Verbindungen der allgemeinen Formel I.24, in welcher die Bedeutungen der Kombinationen aus Z^{1,} Z² und "*" durch die Zeilen der Tabelle A gegeben sind

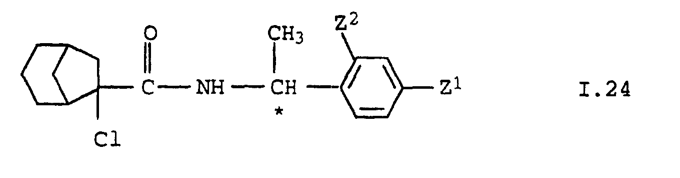

### Tabelle 25:

Verbindungen der allgemeinen Formel I.25, in welcher die Bedeutungen der Kombinationen aus Z^{1,} Z² und "*" durch die Zeilen der Tabelle A gegeben sind

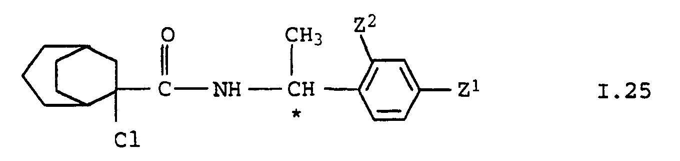

### Tabelle 26:

Verbindungen der allgemeinen Formel I.26, in welcher die Bedeutungen der Kombinationen aus Z¹, Z² und "*" durch die Zeilen der Tabelle A gegeben sind

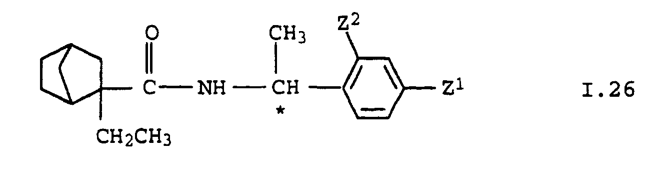

### Tabelle 27:

Verbindungen der allgemeinen Formel I.27, in welcher die Bedeutungen der Kombinationen aus Z^{1,} Z² und "*" durch die Zeilen der Tabelle A gegeben sind

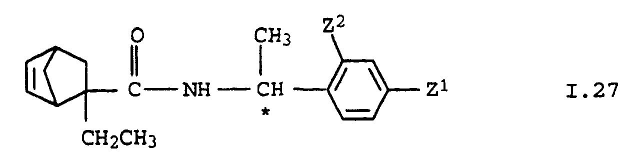

### Tabelle 28:

Verbindungen der allgemeinen Formel I.28, in welcher die Bedeutungen der Kombinationen aus Z^{1,} Z² und "*" durch die Zeilen der Tabelle A gegeben sind

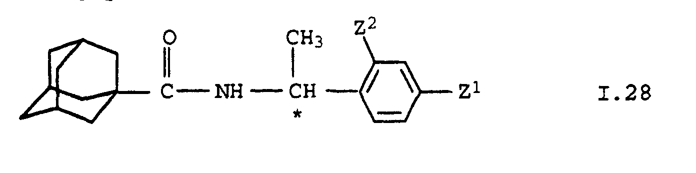

### Tabelle 29:

Verbindungen der allgemeinen Formel I.29, in welcher die Bedeutungen der Kombinationen aus Z^{1,} Z² und "*" durch die Zeilen der Tabelle A gegeben sind

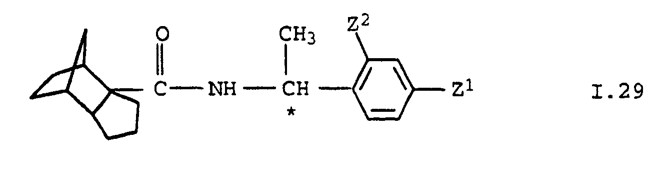

**Tabelle A**

| Nr. | Z¹ | Z² | * |
|---|---|---|---|
| 1 | H | H | R |
| 2 | H | H | S |
| 3 | H | H | rac. |
| 4 | H | Cl | R |
| 5 | H | Cl | S |
| 6 | H | Cl | rac. |
| 7 | H | CH₃ | R |
| 8 | H | CH₃ | S |
| 9 | H | CH3 | rac. |
| 10 | H | OCH₃ | R |
| 11 | H | OCH₃ | S |
| 12 | H | OCH₃ | rac. |
| 13 | H | F | R |
| 14 | H | F | S |
| 15 | H | F | rac. |
| 16 | H | CN | R |
| 17 | H | CN | S |
| 18 | H | CN | rac. |
| 19 | Cl | H | R |
| 20 | Cl | H | S |
| 21 | Cl | H | rac. |
| 22 | Cl | Cl | R |
| 23 | Cl | Cl | S |
| 24 | Cl | Cl | rac. |
| 25 | Cl | CH₃ | R |
| 26 | Cl | CH₃ | S |
| 27 | Cl | CH₃ | rac. |
| 28 | Cl | OCH₃ | R |
| 29 | Cl | OCH₃ | S |
| 30 | Cl | OCH₃ | rac. |
| 31 | Cl | F | R |
| 32 | Cl | F | S |
| 33 | Cl | F | rac. |
| 34 | Cl | CN | R |
| 35 | Cl | CN | S |
| 36 | Cl | CN | rac. |
| 37 | CH₃ | H | R |
| 38 | CH₃ | H | S |
| 39 | CH₃ | H | rac. |
| 40 | CH₃ | Cl | R |
| 41 | CH₃ | Cl | S |
| 42 | CH₃ | Cl | rac. |
| 43 | CH₃ | CH₃ | R |
| 44 | CH₃ | CH₃ | S |
| 45 | CH₃ | CH₃ | rac. |
| 46 | CH₃ | OCH₃ | R |
| 47 | CH₃ | OCH₃ | S |
| 48 | CH₃ | OCH₃ | rac. |
| 49 | CH₃ | F | R |
| 50 | CH₃ | F | S |
| 51 | CH₃ | F | rac. |
| 52 | CH₃ | CN | R |
| 53 | CH₃ | CN | S |
| 54 | CH₃ | CN | rac. |
| 55 | OCH₃ | H | R |
| 56 | OCH₃ | H | S |
| 57 | OCH₃ | H | rac. |
| 58 | OCH₃ | Cl | R |
| 59 | OCH₃ | Cl | S |
| 60 | OCH₃ | Cl | rac. |
| 61 | OCH₃ | CH₃ | R |
| 62 | OCH₃ | CH₃ | S |
| 63 | OCH₃ | CH₃ | rac. |
| 64 | OCH₃ | OCH₃ | R |
| 65 | OCH₃ | OCH₃ | S |
| 66 | OCH₃ | OCH₃ | rac. |
| 67 | OCH₃ | F | R |
| 68 | OCH₃ | F | S |
| 69 | OCH₃ | F | rac. |
| 70 | OCH₃ | CN | R |
| 71 | OCH₃ | CN | S |
| 72 | OCH₃ | CN | rac. |
| 73 | F | H | R |
| 74 | F | H | S |
| 75 | F | H | rac. |
| 76 | F | Cl | R |
| 77 | F | Cl | S |
| 78 | F | Cl | rac. |
| 79 | F | CH₃ | R |
| 80 | F | CH₃ | S |
| 81 | F | CH₃ | rac. |
| 82 | F | OCH₃ | R |
| 83 | F | OCH₃ | S |
| 84 | F | OCH₃ | rac. |
| 85 | F | F | R |
| 86 | F | F | S |
| 87 | F | F | rac. |
| 88 | F | CN | R |
| 89 | F | CN | S |
| 90 | F | CN | rac. |
| 91 | CN | H | R |
| 92 | CN | H | S |
| 93 | CN | H | rac. |
| 94 | CN | Cl | R |
| 95 | CN | Cl | S |
| 96 | CN | Cl | rac. |
| 97 | CN | CH₃ | R |
| 98 | CN | CH₃ | S |
| 99 | CN | CH₃ | rac. |
| 100 | CN | OCH₃ | R |
| 101 | CN | OCH₃ | S |
| 102 | CN | OCH₃ | rac. |
| 103 | CN | F | R |
| 104 | CN | F | S |
| 105 | CN | F | rac. |
| 106 | CN | CN | R |
| 107 | CN | CN | S |
| 108 | CN | CN | rac. |
| (* = Konfiguration des jeweils in den Formeln I.1 bis I.29 mit "*" gekennzeichneten Atoms; R = R-Konfiguration; S = S-Konfiguration; rac. = racemisch) | | | |

Die neuen Verbindungen I eignen sich zur Bekämpfung von Schadpilzen.

Sie können in Abhängigkeit von ihren chemischen und physikalischen Eigenschaften mit üblichen, also dem Fachmann geläufigen, Formulierungshilfsmitteln formuliert werden. Die so hergestellten Produkte werden als "Mittel" bezeichnet.

Geeignete Formulierungshilfsmittel sind z.B. feste oder flüssige Trägerstoffe, oberflächenaktive Mittel und Haftmittel.

Unter flüssigen Trägerstoffen werden flüssige Lösungsmittel wie Wasser und organische Lösungsmittel verstanden, wobei letztere vor allem bei Verwendung von Wasser als Lösungsmittel die Funktion eines Hilfslösungsmittels haben. Als organische Lösungsmittel können verwendet werden: Aromaten wie Xylol, Toluol und Alkylnaphthaline, chlorierte Aromaten oder chlorierte aliphatische Kohlenwasserstoffe wie Chlorbenzole, Chlorethylene und Methylenchlorid, aliphatische Kohlenwasserstoffe wie Cyclohexan und Paraffine, z.B. Mineralölfraktionen, Alkohole wie Butanol, iso-Butanol, Cyclohexanol und Glykol sowie die zugehörigen Ether und Ester, Ketone wie Aceton, Methylethylketon, Methyl-iso-butylketon und Cyclohexanon, aprotisch dipolare Lösungsmittel wie Dimethylformamid, N-Methyl-2-pyrrolidon und Dimethylsulfoxid.

Als feste Trägerstoffe kommen beispielsweise in Betracht: Natürliche Gesteinsmehle und Mineralerden wie Kieselsäuren, Silicate, Kaoline, Tonerden, Bolus, Löß, Talkum, Kreide, Kalkstein, Kalk, Dolomit, Magnesiumoxid, Quarz, Attapulgit, Montmorillonit und Diatomeenerde; synthetische Gesteinsmehle wie hochdisperse Kieselsäure oder Mehle von synthetischem Aluminiumoxid und von synthetischen Silikaten. Insbesondere für Granulate geeignete feste Trägerstoffe sind beispielsweise: Gebrochene und fraktionierte natürliche Gesteine wie Calcit, Marmor, Bims, Sepiolith; synthetische Granulate aus anorganischen und organischen Mehlen; Granulate aus organischem Material wie Sägemehl, Kokosnußschalen, Maiskolben oder Tabakstengel.

Geeignete oberflächenaktive Mittel sind nichtionogene und anionische Emulgiermittel/schaumerzeugende Mittel und Dispergiermittel:
- Fettsäure-Polyoxyethylenester wie Laurylalkohol-Polyoxyethylenetheracetat,
- Alkyl-Polyoxyethylen- oder -Polyoxypropylenether etwa von iso-Tridecylalkohol und Fettalkohol-Polyoxyethylenether,
- Alkylarylalkohol-Polyoxyethylenether wie Octylphenol-Polyoxyethylenether,
- Tributylphenol-Polyoxyethylenether,
- ethoxyliertes iso-Octyl-, Octyl- oder Nonylphenol oder Rizinusöl,
- Sorbitester,
- Arylsulfonsäuren, Alkylsulfonsäuren, Alkylschwefelsäuren,
- Alkali-, Erdalkali- und Ammoniumsalze von Arylsulfonsäuren, z.B. Lignin-, Phenol-, Naphthalin- und Dibutylnaphthalinsulfonsäure, Alkylsulfonsäuren, Alkylarylsulfonsäuren, Alkyl-, Laurylether- und Fettalkoholschwefelsäuren, Fettsäuren, sulfatierten Hexa-, Hepta- und Octadecanolen und Fettalkoholglykolethern,
- Kondensationsprodukte von sulfoniertem Naphthalin und seiner Derivate mit Formaldehyd,
- Kondensationsprodukte von Naphthalinsulfonsäuren mit Phenol und Formaldehyd,
- Eiweißhydrolysate und
- insbesondere als Dispergiermittel: Lignin-Sulfitablaugen und Methylcellulose.

Als Haftmittel eignen sich beispielsweise: Carboxymethylcellulose; natürliche und synthetische pulverige, körnige oder latexförmige Polymere wie Gummiarabicum, Polyvinylalkohol, Polyvinylacetat, natürliche Phospholipide wie Kephaline und Lecithine, synthetische Phospholipide.

Weiterhin können die Mittel einen oder mehrere Vertreter der folgenden Stoffgruppen enthalten: Farbstoffe, andere bekannte Wirkstoffe, Spurennährstoffe und weitere Additive.

Als Farbstoffe kommen z.B. anorganische Pigmente wie Eisenoxid, Titanoxid, Ferrocyanblau, ferner organische Pigmente wie Alizarin-, Azo- und Metallphthalocyaninfarbstoffe in Betracht. Unter anderen bekannten Wirkstoffen sind etwa andere Fungizide sowie Insektizide, Akarizide, Herbizide und Wachstumsregulatoren zu verstehen. Spurennährstoffe sind beispielsweise Salze von Eisen, Mangan, Bor, Kupfer, Kobalt, Molybdän und Zink. Als weitere Additive sind etwa mineralische und vegetabile Öle geeignet.

Die Mittel können darüberhinaus mit sonstigen, praktisch bedeutsamen Mischungspartnern wie Düngemittel oder sonstige fertige wirkstoffhaltige Mittel vermischt sein.

Die Herstellung der Mittel erfolgt in an sich bekannter Weise, nämlich in Abhängigkeit von den chemischen und physikalischen Eigenschaften der eingesetzten Stoffe z.B. durch Mischen, gemeinsames Vermahlen, Aufsprühen, Extrudieren, Granulieren oder Auflösen in Wasser, letzteres ggf. unter Zuhilfenahme eines organischen Lösungsmittels. Pulver, Streu- und Stäubemittel sind z.B. durch Mischen oder gemeinsames Vermahlen der Verbindungen I mit einem festen Trägerstoff erhältlich.

Bei den Mitteln handelt es sich in Abhängigkeit von den eingesetzten Stoffen z.B. um Lösungen, Emulsionen, Suspensionen, Pulver, Schäume, Pasten, Granulate, Aerosole oder Feinstverkapselungen in polymeren Stoffen oder in Saatgut-Hüllmassen.

Zur Anwendung werden die für den Handel in der Regel als Konzentrate vorliegenden Mittel gegebenenfalls wie üblich aufgelöst, verdünnt usw., bei Spritzpulvern, wasserdispergierbaren Granulaten, emulgierbaren Konzentraten, Dispersionen und teilweise auch bei Mikrogranulaten normalerweise unter Verwendung von Wasser. Staubförmige und granulierte Zubereitungen sowie versprühbare Lösungen werden vor der Anwendung meist nicht mehr mit weiteren inerten Stoffen verdünnt.

Die Ausbringung der Mittel erfolgt in an sich bekannter Weise, etwa durch Versprühen, Vernebeln, Verstäuben, Verstreuen oder Gießen. Die Pflanzen werden in der Regel mit den Mitteln besprüht oder bestäubt. Alternativ oder zusätzlich behandelt man die Samen der Pflanzen in an sich bekannter Weise.

Beispiele für solche Zubereitungen sind:
- 16.: eine Lösung aus 90 Gew.-Teilen einer erfindungsgemäßen Verbindung I und 10 Gew.-Teilen N-Methyl-2-pyrrolidon, die zur Anwendung in Form kleinster Tropfen geeignet ist;
- 17.: eine Mischung aus 20 Gew.-Teilen einer erfindungsgemäßen Verbindung I, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen des Calciumsalzes der Dodecylbenzolsulfonsäure, 5 Gew.-Teilen des Anlagerungsproduktes von 40 Mol Ethylenoxid an 1 Mol Ricinusöl: durch feines Verteilen der Lösung in Wasser erhält man eine Dispersion;
- 18.: eine wäßrige Dispersion aus 20 Gew.-Teilen einer erfindungsgemäßen Verbindung I, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
- 19.: eine wäßrige Dispersion aus 20 Gew.-Teilen einer erfindungsgemäßen Verbindung I, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
- 20.: eine in einer Hammermühle vermahlene Mischung aus 80 Gew.-Teilen einer erfindungsgemäßen Verbindung I, 3 Gew.-Teilen des Natriumsalzes der Diisobutylnaphtalin-1-sulfonsäure, 10 Gew.-Teilen des Natriumsalzes einer Ligninsulfonsäure aus einer Sulfitablauge und 7 Gew.-Teilen pulverförmigem Kieselsäuregel: durch feines Verteilen der Mischung in Wasser erhält man eine Spritzbrühe;
- 21.: eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
- 22.: eine innige Mischung aus 30 Gew.-Teilen einer erfindungsgemäßen Verbindung I, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
- 23.: eine stabile wäßrige Dispersion aus 40 Gew.-Teilen einer erfindungsgemäßen Verbindung I, 10 Gew.-Teilen des Natriumsalzes eines Phenolsulfonsäure-Harnstoff-Formaldehyd-Kondensates, 2 Gew.-Teilen Kieselgel und 48 Gew.-Teilen Wasser, die weiter verdünnt werden kann;
- 24.: eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkoholpolyglykolether, 20 Gew.-Teilen des Natriumsalzes eines Phenolsulfonsäure-Harnstoff-Formaldehyd-Kondensates und 68 Gew.-Teilen eines paraffinischen Mineralöls.

Werden die Verbindungen I als solche appliziert, so kommt es vor allem auf deren feine Verteilung an.

Die Verbindungen I und die erfindungsgemäßen Mittel zeichnen sich durch eine hervorragende Wirksamkeit gegen ein breites Spektrum von Schadpilzen (pflanzenpathogene Pilze), insbesondere aus der Klasse der
- Ascomyceten,
- Basidiomyceten,
- Deuteromyceten und
- Phycomyceten
aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen sowie an den Samen dieser Pflanzen.

Die Verbindungen I und die erfindungsgemäßen Mittel werden angewendet, indem man die Schadpilze, deren Lebensraum oder die vor Pilzbefall zu schützenden Saatgüter, Pflanzen, Flächen, Materialien oder Räume mit einer fungizid wirksamen Menge der Mittel oder der Verbindungen I behandelt. Die Anwendung kann vor oder nach dem Befall durch die Pilze erfolgen.

Speziell eignen sich die erfindungsgemäßen Mittel und die Verbindungen I zur Bekämpfung folgender Pflanzenkrankheiten:

Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle, Reis und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Pseudoperonospora-Arten in Hopfen und Gurken, Alternaria-Arten an Gemüse und Obst. Die Bekämpfung von Botrytis mittels der erfindungsgemäßen Mittel oder der Verbindungen I ist bevorzugt.

Auch im Materialschutz (Holzschutz) können die erfindungsgemäßen Mittel oder die Verbindungen I eingesetzt werden, z.B. gegen Paecilomyces variotii.

Der Gehalt an den Verbindungen I in den erfindungsgemäßen Mitteln beträgt im allgemeinen zwischen 0,1 und 95, vorzugsweise zwischen 0,5 und 90 Gew.-%.

Die Aufwandmengen an den Verbindungen I liegen je nach Art des gewünschten Effektes zwischen 0,01 und 2,0 kg pro ha.

Bei der Saatgutbehandlung werden im allgemeinen 0,001 bis 50, vorzugsweise 0,01 bis 10 g einer Verbindung I je Kilogramm Saatgut benötigt.

In den erfindungsgemäßen Mittel können die Verbindungen I auch zusammen mit anderen im Planzenschutz gebräuchlichen Wirkstoffen, z.B. Herbiziden, Insektiziden, Wachstumsregulatoren, Fungiziden oder auch Düngemitteln, vorliegen. Beim Vermischen mit weiteren Fungiziden erhält man dabei in vielen Fällen eine Vergrößerung des fungiziden Wirkungsspektrums.

Die folgende Liste von fungiziden Wirkstoffen, mit denen die Verbindungen I gemeinsam angewendet werden können, soll die Kombinationsmöglichkeiten erläutern.
Schwefel, Dithiocarbamate und deren Derivate wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)disulfid;
Nitroderivate wie Dinitro-(1-methylheptyl)phenylcrotonat, 2-sec.-Butyl-4,6-dinitrophenyl-3,3-dimethylacrylat, 2-sec.-Butyl-4,6-dinitrophenyl-iso-propylcarbonat, 5-Nitro-iso-phthalsäure-di-isopropylester;
heterocyclische Substanzen wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)phosphinyl]-3-phenyl-1,2,4-triazol, 2,3-Dicyano-1,4-dithioanthrachinon, 2-Thio-1,3-dithiolo-[4,5-b]-chinoxalin, 1-(Butylcarbamoyl)-2-benzimidazol-carbaminsäuremethylester, 2-Methoxycarbonylamino-benzimidazol, 2-(Furyl-(2))benzimidazol, 2-(Thiazolyl-(4))benzimidazol, N-(1,1,2,2-Tetrachlorethylthio)tetrahydrophthalimid, N-Trichlormethylthio-tetrahydrophthalimid, N-Trichlor-methylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlor-ethylacetal, Piperazin-1,4-diylbis-(1-(2,2,2-trichlor-ethyl)formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecylmorpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-ylethyl]-1H-1,2,4-triazol, N-(n-Propyl)-N-(2,4,6-trichlor-phenoxyethyl)-N'-imidazol-yl-harnstoff, 1-(4-Chlorphenoxy)-3,3-dimethyl-1-(1H-1,2,4-triazol-1-yl)-2-butanon, (2-Chlorphenyl)-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)benzol, 1,2-Bis-3-methoxycarbonyl-2-thioureido)benzol, [2-(4-Chlorphenyl)ethyl]-(1,1-dimethylethyl)-1H-1,2,4-triazol-1-ethanol, 1-[3-(2-Chlorphenyl)-1-(4-fluorphenyl)oxiran-2-yl-methyl]-1H-1,2,4-triazol sowie
verschiedene Fungizide wie Dodecylguanidinacetat, 3-[3-(3,5-Dimethyl-2-oxycyclohexyl)-2-hydroxyethyl)]glutarimid, Hexachlorbenzol, DL-Methyl-N-(2,6-dimethyl-phenyl)-N-furoyl(2)alaninat, DL-N-(2,6-Dimethyl-phenyl)-N-(2'-methoxyacetyl)alanin-methylester, N-(2,6-Dimethylphenyl)-N-chloracetyl-D,L-2-aminobutyrolacton, DL-N-(2,6-Dimethylphenyl)-N-(phenylacetyl)alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorhenyl)-1-iso-propylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)pentyl]-1H-1,2,4-triazol, 2,4-Difluor-a-(1H-1,2,4-triazolyl-l-methyl)benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)methylsilyl)methyl)-1H-1,2,4-triazol.
Strobilurine wie Methyl-E-methoximino-[a-(o-tolyloxy)-o-tolyl]acetat, Methyl-E-2-{2-[6-(2-cyanophenoxy)pyrimidin-4-yloxy]-phenyl}-3-methoxyacrylat, Methyl-E-methoximino-[a-(2-phenoxyphenyl)]acetamid, Methyl-E-methoximino-[a-(2,5-dimethylphenoxy)-o-tolyl]acetamid.
Anilinopyrimidine wie N-(4,6-Dimethylpyrimidin-2-yl)anilin, N-[4-Methyl-6-(1-propinyl)pyrimidin-2-yl]anilin, N-(4-Methyl-6-cyclopropyl-pyrimidin-2-yl)anilin.
Phenylpyrrole wie 4-(2,2-difluor-1,3-benzodioxol-4-yl)pyrrol-3-carbonitril.
Zimtsäureamide wie 3-(4-Chlorphenyl)-3-(3,4-dimethoxyphenyl)acrylsäuremorpholid.
(2RS,3SR)-1-[3-(2-Chlorphenyl)-2-[4-fluorphenyl]oxiran-2-ylmethyl]-1H-1,2,4-triazol.

### Synthesebeispiele

### Beispiel 1

### 2-Methyl-Bicyclo[2.2.1]hept-5-en-2-carboxy-(1-(p-Chlorphenyl)ethylamid

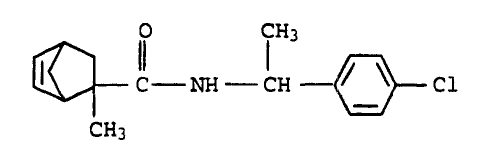

Zu der Lösung von 0,53 g (3,46 mmol) 2-Methylbicyclo[2.2.1]hept-5-en-2-carbonsäure und 0,54 g (3,46 mmol) racemisches 1-Amino-1-(p-Chlorphenyl)ethan in 50 ml Dichlormethan wurden bei 0 °C 0,56 g (3,46 mmol) Cyanphosphorsäurediethylester und 0,77 g (7,61 mmol) Triethylamin zugegeben. Es wurde eine Stunde unter Eiskühlung und 15 Stunden bei 20 °C nachgerührt. Anschließend wurde die Lösung nacheinander mit dem gleichen Volumen an 2n Natronlauge, Wasser, 10%-iger Salzsäure und nochmals Wasser gewaschen, getrocknet und eingeengt. Es verblieben 0,8 g (2,76 mmol) der Titelverbindung (Fp. 118-22 °C, Verbindung 1.7 in Tabelle E1).

Die in den nachstehenden Synthesebeispielen wiedergegebenen Vorschriften können unter Abwandlung der Ausgangsverbindungen zur Gewinnung weiterer Vertreter der Verbindungen I benutzt werden. Die physikalischen Daten der demgemäß hergestellten Produkte sind in den anschließenden Tabellen wiedergegeben.

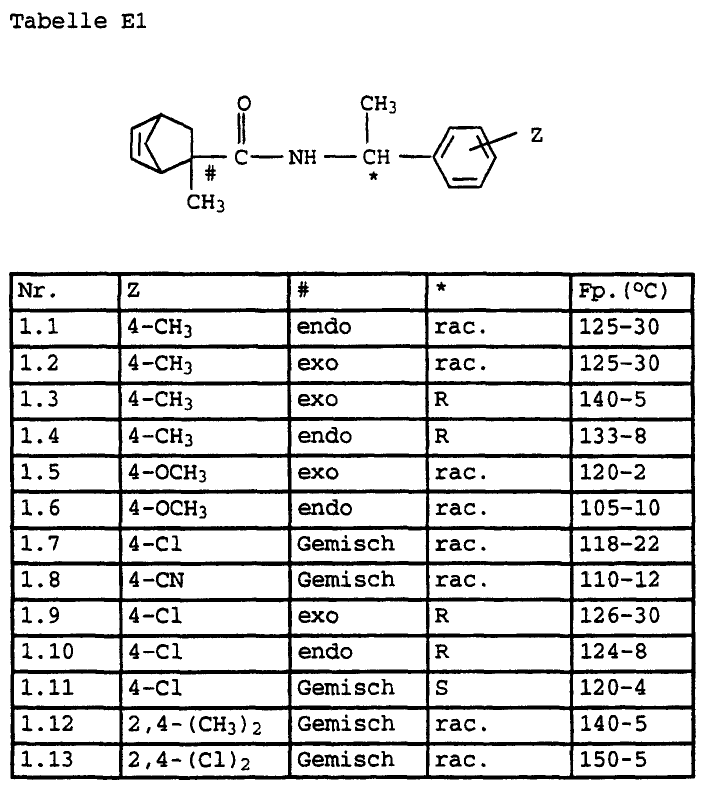

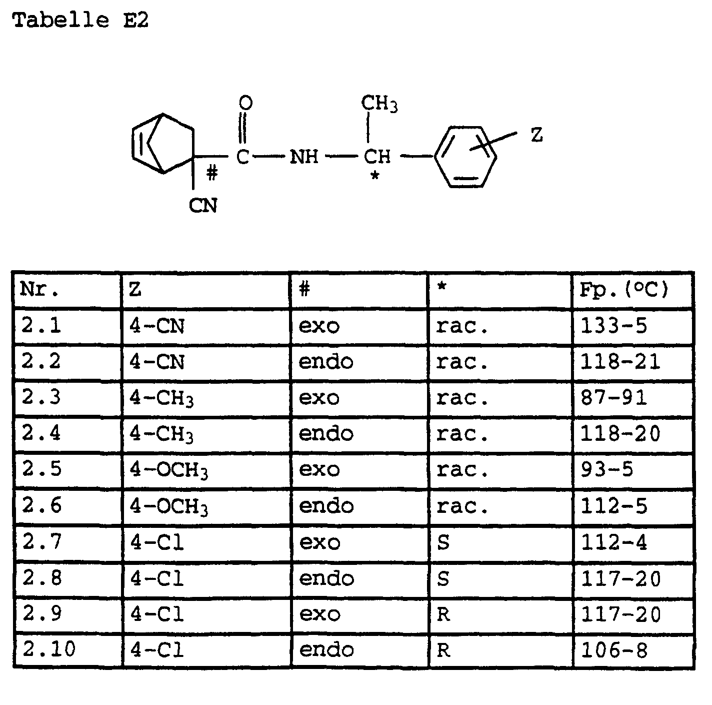

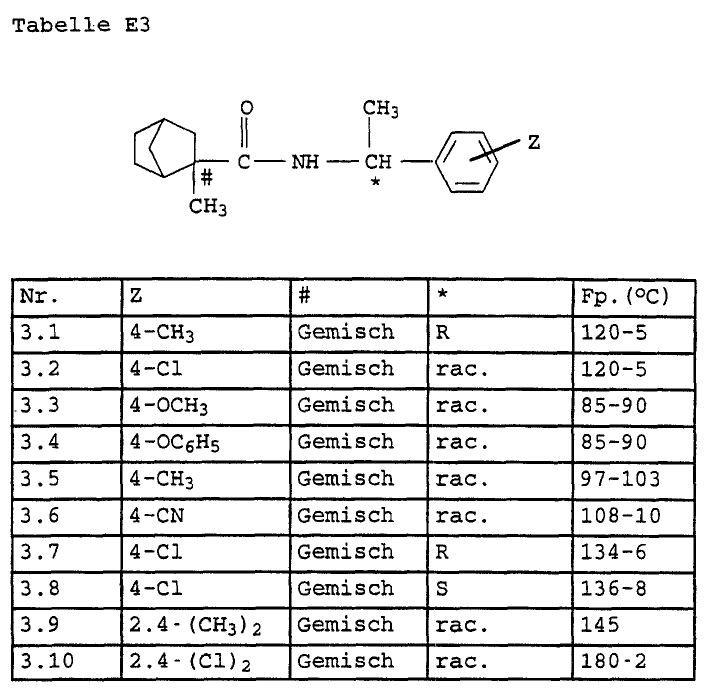

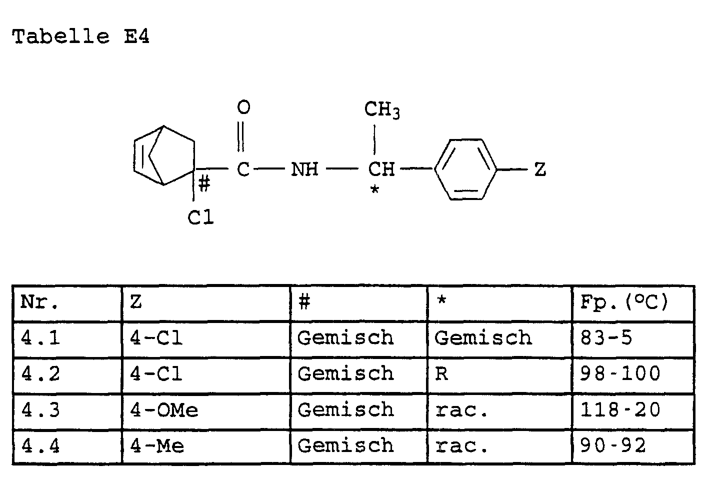

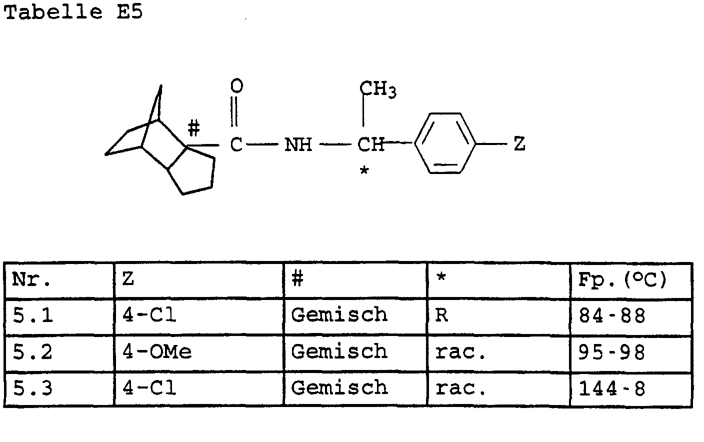

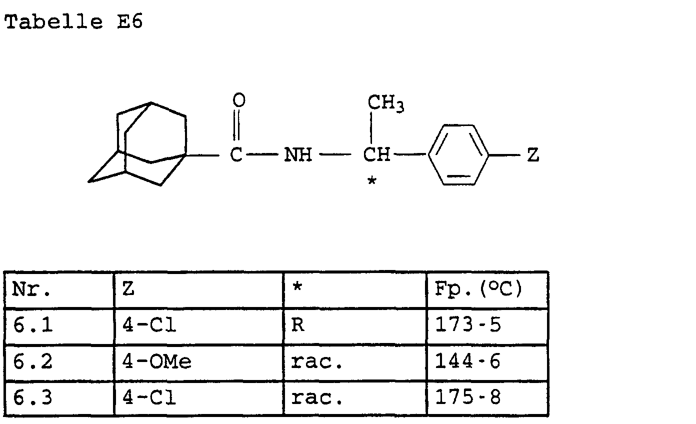

### Anwendungsbeispiele

Die fungizide Wirkung der Verbindungen der allgemeinen Formel I ließ sich durch folgende Versuche zeigen:

Die Wirkstoffe wurden als 20 gew.-%ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und enstprechend der gewünchten Konzentration mit Wasser verdünnt.

### Wiksamkeit gegen Pyricularia oryzae

Blätter von in Töpfen gewachsenen Reiskeimligen der Sorte "Tai-Nong 67" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inokuliert. Anschließend wurden die Versuchspflanzen in Klimakammern bei 22 - 24 °C und 95 - 99 % relativer Luftfeuchtigkeit aufgestellt. Nach 6 Tagen wurde das Ausmaß des Krankheitsbefalls ermittelt.

In diesem Versuch wurden die Wirkstoffe 1.3, 1.4, 1.7, 1.9 (Tabelle E1) sowie 3.1, 3.7 (Tabelle E3) jeweils einzeln in Form einer wäßrigen Spritzbrühe mit einem Wirkstoffgehalt von 250 ppm angewandt.

Der Befall der Blätter mit Pyricularia oryzae bei Versuchsende lag bei 0 bis 5 % der Blattfläche, wohingegen unbehandelte Blätter zu 70 % befallen waren.

## Patentansprüche

1. Carbonsäureamide der Formel I 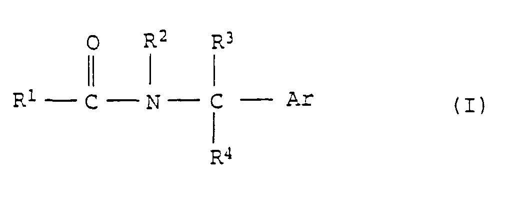 in der die Substituenten die folgenden Bedeutungen haben:
R¹ C₆-C₁₅-Bicycloalkyl, Tricyclo [3.3.1.1.^{3,7}]decan, Tricyclo [5.2.1.0^{2,6}] decan oder C₇-C₁₅-Bicycloalkenyl, wobei diese Reste partiell oder vollständig halogeniert sein können und/oder, sofern sie nicht vollständig halogeniert sind, eine oder unabhängig voneinander zwei, drei, vier oder fünf der folgenden Gruppen tragen können: Cyano, C₁-C₄-Alkyl, C₁-C₄-Halogenalkyl, C₁-C₄-Alkoxy, C₁-C₄-Halogenalkoxy und Aryl, wobei das Aryl partiell oder vollständig halogeniert sein und/oder einen oder unabhängig voneinander zwei oder drei der folgenden Substituenten tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio;
R² Wasserstoff,
R³ Wasserstoff,
Ar Aryl oder Heteroaryl, wobei diese Reste eine oder unabhängig voneinander zwei oder drei der folgenden Gruppen tragen können: Halogen, Cyano, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Alkoxycarbonyl, Aryl, Aryloxy und Heteroaryl, wobei in diesen Gruppen die Ringe ihrerseits einen oder unabhängig voneinander zwei oder drei der folgenden Substituenten tragen können: Halogen, Cyano, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio und C₁-C₄-Alkoxycarbonyl,
R⁴ C₁-C₈-Alkyl, welches partiell oder vollständig halogeniert sein und/oder eine oder zwei der folgenden Gruppen tragen kann: C₁-C₄-Alkoxy, C₁-C₄-Halogenalkoxy, C₁-C₄-Alkylthio, C₃-C₇-Cycloalkyl, C₅-C₇-Cycloalkenyl, wobei die cyclischen Gruppen ihrerseits ein oder unabhängig voneinander zwei oder drei Halogenatome, C₁-C₃-Alkylgruppen und/oder C₁-C₃-Alkoxygruppen tragen können und Aryl, wobei das Aryl partiell oder vollständig halogeniert sein und/oder einen oder unabhängig voneinander zwei oder drei der folgenden Substituenten tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio oder
C₃-C₆-Cycloalkyl, C₃-C₆-Cycloalkenyl oder Heterocyclyl, wobei diese Reste partiell oder vollständig halogeniert sein und/oder eine oder unabhängig voneinander zwei oder drei der folgenden Gruppen tragen können: Cyano, C₁-C₆-Alkyl, C₁-C₆-Halogenalkyl, C₂-C₆-Alkenyl, C₁-C₆-Alkoxy und C₁-C₆-Alkylthio;
mit der Maßgabe, daß Ar nicht unsubstituiertes Phenyl ist, wenn R¹ Tricyclo[3.3.1.1^{3,7}] decan bedeutet,
ausgenommen 2-Cyano-N-[1-(1-naphthyl)ethyl]-3-phenylbicyclo[2.2.1]hept-5-en-2-carboxamid, Endo-bicyclo[3.3.1]non-6-en-3-carboxy-(1-phenyl)ethylamid, (5R,6S)-6-Methylbicyclo[2.2.1]hept-2-en-5-carboxy-R-1-(1-naphthyl)ethylamid, 2,5,5,8a-Tetramethyldecahydronaphthalin-1-carbonsäure(1-phenyl)ethylamid.

2. Carbonsäureamide gemäß Anspuch 1, in denen das Kohlenstoffatom, das die Gruppen R³ und R⁴ trägt, R-Konfiguration hat.

3. Carbonsäureamide gemäß Anspuch 1, in denen R⁴ für C₁-C₄-Alkyl steht.

4. Carbonsäureamide gemäß Anspuch 3, in denen R⁴ für Methyl steht.

5. Carbonsäureamide gemäß Anspuch 1, in denen Ar für Phenyl steht, das in 2-Position, 2- und 4-Position oder 4-Position substituiert ist.

6. Carbonsäureamide gemäß Anspuch 1, in denen R¹ für ein gegebenenfalls substituiertes Bicycloalkyl mit 6 - 10 Kohlenstoffatomen oder für ein gegebenenfalls substituiertes Bicycloalkenyl mit 7 - 10 Kohlenstoffatomen steht.

7. Verfahren zur Herstellung der Verbindungen I gemäß den Ansprüchen 1-6, dadurch gekennzeichnet, daß man ein Amin der Formel II 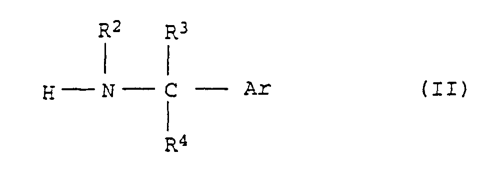 in Gegenwart einer Base mit einer carboxyaktivierten Carbonsäure der Formel III
R¹-COX (III)
in dem X für Halogen oder eine bei Acylierungsreaktionen gebräuchliche Abgangsgruppe steht, umsetzt.

8. Zur Bekämpfung von Schadpilzen geeignete Mittel, enthaltend eine wirksame Menge einer Verbindung der Formel I gemäß den Ansprüchen 1-6 und mindestens ein übliches Formulierungshilfsmittel.

9. Verfahren zur Bekämpfung von Schadpilzen, dadurch gekennzeichnet, daß man die Schadpilze, deren Lebensraum oder die von ihnen freizuhaltenden Pflanzen, Flächen, Materialien oder Räume mit einer fungizid wirksamen Menge einer Verbindung der Formel I gemäß den Ansprüchen 1-6 behandelt.

10. Verwendung der Verbindungen I gemäß den Ansprüchen 1-6 zur Bekämpfung von Schadpilzen.

## Claims

1. A carboxamide of the formula I 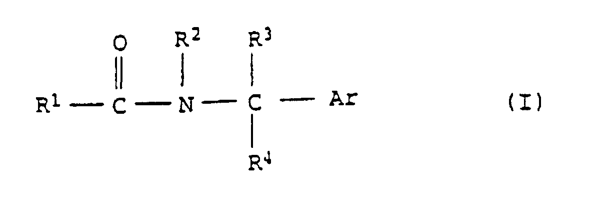 in which the substituents are as defined below:
R¹ is C₆-C₁₅-bicycloalkyl, tricyclo [3.3.1.1.^{3,7}] decane, tricycle [5.2.1.0.^{2,6}] decane or C₇-C₁₅-bicycloalkenyl, where these radicals may be partially or fully halogenated and/or may, if they are not completely halogenated, carry one or, independently of one another, two, three, four or five of the following groups: cyano, C₁-C₄-alkyl, C₁-C₄-haloalkyl, C₁-C₄-alkoxy, C₁-C₄-haloalkoxy and aryl, where the aryl may be partially or fully halogenated and/or may carry one or, independently of one another, two or three of the following substituents: nitro, cyano, C₁-C₄-alkyl, C₁-C₄-haloalkyl, C₁-C₄-alkoxy, C₁-C₄-haloalkoxy and C₁-C₄-alkylthio;
R² is hydrogen,
R³ is hydrogen,
Ar is aryl or hetaryl, where these radicals may carry one or, independently of one another, two or three of the following groups: halogen, cyano, C₁-C₄-alkyl, C₁-C₄-alkoxyalkyl, C₁-C₄-haloalkyl, C₁-C₄-alkoxy, C₁-C₄-haloalkoxy, C₁-C₄-alkylthio, C₁-C₄-alkoxycarbonyl, aryl, aryloxy and hetaryl, where the rings in these groups may for their part carry one or, independently of one another, two or three of the following substituents: halogen, cyano, C₁-C₄-alkyl, C₁-C₄-alkoxyalkyl, C₁-C₄-haloalkyl, C₁-C₄-alkoxy, C₁-C₄-haloalkoxy, C₁-C₄-alkylthio and C₁-C₄-alkoxycarbonyl.
R⁴ is C₁-C₈-alkyl which may be partially or fully halogenated and/or may carry one or two of the following groups: C₁-C₄-alkoxy, C₁-C₄-haloalkoxy, C₁-C₄-alkylthio, C₃-C₇-cycloalkyl, C₅-C₇-cycloalkenyl, where the cyclic groups for their part may carry one or, independently of one another, two or three halogen atoms, C₁-C₃-alkyl groups and/or C₁-C₃-alkoxy groups and aryl, where the aryl may be partially or fully halogenated and/or may carry one or, independently of one another, two or three of the following substituents: nitro, cyano, C₁-C₄-alkyl, C₁-C₄-haloalkyl, C₁-C₄-alkoxy, C₁-C₄-haloalkoxy and C₁-C₄-alkylthio or C₃-C₆-cycloalkyl, C₃-C₆-cycloalkenyl or heterocyclyl, where these radicals may be partially or fully halogenated and/or may carry one or, independently of one another, two or three of the following groups: cyano, C₁-C₆-alkyl, C₁-C₆-haloalkyl, C₂-C₆-alkenyl, C₁-C₆-alkoxy and C₁-C₆-alkylthio;
with the proviso that Ar is not unsubstituted phenyl if R¹ is tricyclo [3.3.1.1^{3,7}]decane,
except for 2-cyano-N-[1-(1-naphthyl)ethyl]-3-phenylbicyclo[2.2.1]hept-5-ene-2-carboxamide, (1-phenyl)ethyl endobicyclo[3.3.1]non-6-ene-3-carboxamide, R-[1-(1-naphthyl)ethyl]-(5R,6S)-6-methylbicyclo[2.2.1]hept-2-ene-5-carboxamide, (1-phenyl)ethyl-2,5,5,8a-tetramethyldecahydronaphthalene-1-carboxamide.

2. A carboxamide as claimed in claim 1 in which the carbon atom which carries groups R³ and R⁴ has the R configuration.

3. A carboxamide as claimed in claim 1 in which R⁴ is C₁-C₄-alkyl.

4. A carboxamide as claimed in claim 3 in which R⁴ is methyl.

5. A carboxamide as claimed in claim 1 in which Ar is phenyl which is substituted in the 2 position, 2 and 4 position or 4 position.

6. A carboxamide as claimed in claim 1 in which R¹ is unsubstituted or substituted bicycloalkyl having 6-10 carbon atoms or is unsubstituted or substituted bicycloalkenyl having 7-10 carbon atoms.

7. A process for preparing the compounds I as claimed in any of claims 1-6, which comprises reacting an amine of the formula II 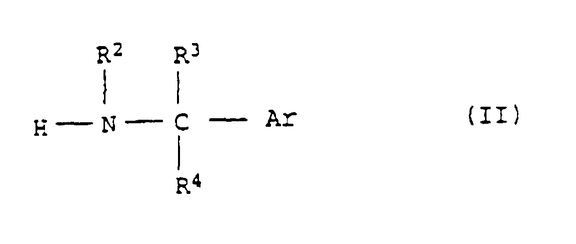 in the presence of a base with a carboxy-activated carboxylic acid of the formula III
R¹-COX (III)
in which X is halogen or a leaving group which is customary for acylation reactions.

8. A composition suitable for controlling harmful fungi, comprising an effective amount of a compound of the formula I as claimed in any of claims 1-6 and at least one customary formulation auxiliary.

9. A method for controlling harmful fungi, which comprises treating the fungi, the habitat or the plants, areas, materials or spaces to be kept free from them with a fungicidally effective amount of a compound of the formula I as claimed in any of claims 1-6.

10. The use of the compounds I as claimed in any of claims 1-6 for controlling harmful fungi.

## Revendications

1. Amides d'acides carboxyliques de formule I 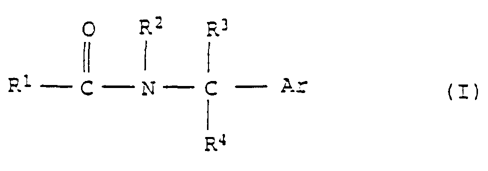 dans laquelle les symboles ont les significations suivantes :
R¹ : un groupe bicycloalkyle en C6-C15, tricyclo[3.3.1.1.^{3,7}]décane, tricyclo[5.2.1.0^{2,6}]décane ou bicycloalcényle en C7-C15, ces groupes pouvant être halogénés en totalité ou en partie et/ou, lorsqu'ils ne sont pas halogénés en totalité, peuvent porter un ou, indépendamment les uns des autres, deux, trois, quatre ou cinq des substituants suivants : cyano, alkyle en C1-C4, halogénoalkyle en C1-C4, alcoxy en C1-C4, halogénoalcoxy en C1-C4, aryle, ce dernier pouvant être halogéné en totalité ou en partie et/ou pouvant porter un ou, indépendamment les uns des autres, deux ou trois des substituants suivants : nitro, cyano, alkyle en C1-C4, halogénoalkyle en C1-C4, alcoxy en C1-C4, halogénoalcoxy en C1-C4 et alkylthio en C1-C4 ;
R² : l'hydrogène ;
R³ : l'hydrogène ;
Ar : un groupe aryle ou hétéroaryle, ces groupes pouvant porter un ou, indépendamment les uns des autres, deux ou trois des substituants suivants : halogéno, cyano, alkyle en C1-C4, alcoxyalkyle en C1-C4, halogénoalkyle en C1-C4, alcoxy en C1-C4, halogénoalcoxy en C1-C4, alkylthio en C1-C4, alcoxycarbonyle en C1-C4, aryle, aryloxy et hétéroaryle, les cycles de ces groupes pouvant eux-mêmes porter un ou, indépendamment les uns des autres, deux ou trois des substituants suivants : halogéno, cyano, alkyle en C1-C4, alcoxyalkyle en C1-C4, halogénoalkyle en C1-C4, alcoxy en C1-C4, halogénoalcoxy en C1-C4, alkylthio en C1-C4 et alcoxycarbonyle en C1-C4,
R⁴ : un groupe alkyle en C1-C8 qui peut être halogéné en totalité ou en partie et/ou peut porter un ou deux des substituants suivants : alcoxy en C1-C4, halogénoalcoxy en C1-C4, alkylthio en C1-C4, cycloalkyle en C3-C7, cycloalcényle en C5-C7, les groupes cycliques pouvant eux-mêmes porter un ou, indépendamment les uns des autres, deux ou trois atomes d'halogènes, groupes alkyle en C1-C3 et/ou alcoxy en C1-C3, et aryle, ce dernier pouvant être halogéné en totalité ou en partie et/ou pouvant porter un ou, indépendamment les uns des autres, deux ou trois des substituants suivants : nitro, cyano, alkyle en C1-C4, halogénoalkyle en C1-C4, alcoxy en C1-C4, halogénoalcoxy en C1-C4 et alkylthio en C1-C4 ou bien
un groupe cycloalkyle en C3-C6, cycloalcényle en C3-C6 ou hétérocyclyle, ces groupes pouvant être halogénés en totalité ou en partie et/ou pouvant porter un ou, indépendamment les uns des autres, deux ou trois des substituants suivants : cyano, alkyle en C1-C6, halogénoalkyle en C1-C6, alcényle en C2-C6, alcoxy en C1-C6 et alkylthio en C1-C6 ;
sous réserve que Ar ne peut représenter un groupe phényle non substitué lorsque R¹ représente le tricyclo[3.3.1.1^{3,7}]décane,
à l'exception du 2-cyano-N-[1-(1-naphtyl)éthyl]-3-phényl-bicyclo[2.2.1]hept-5-ène-2-carboxamide, de l'endo-bicyclo[3.3.1]non-6-ène-3-carboxy-(1-phényl)éthylamide, du (5R, 6S)-6-méthylbicyclo[2.2.1]hept-2-ène-5-carboxy-R-1-(1-naphtyl)éthylamide, du (1-phényl)éthylamide de l'acide 2,5,5,8a-tétraméthyldécahydronaphtalène-1-carboxylique.

2. Amides d'acides carboxyliques selon la revendication 1, dans lesquels l'atome de carbone portant les groupes R³ et R⁴ est en configuration R.

3. Amides d'acides carboxyliques selon la revendication 1, dans lesquels R⁴ représente un groupe alkyle en C1-C4.

4. Amides d'acides carboxyliques selon la revendication 3, dans lesquels R⁴ représente un groupe méthyle.

5. Amides d'acides carboxyliques selon la revendication 1, dans lesquels Ar représente un groupe phényle substitué en position 2, dans les positions 2 et 4 ou en position 4.

6. Amides d'acides carboxyliques selon la revendication 1, dans lesquels R¹ représente un groupe bicycloalkyle en C6-C10 éventuellement substitué ou un groupe bicycloalcényle en C7-C10 éventuellement substitué.

7. Procédé de préparation des composés I selon les revendications 1 à 6, caractérisé par le fait que l'on fait réagir une amine de formule II 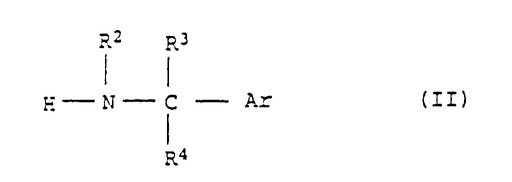 en présence d'une base, avec un acide carboxylique à groupe carboxy activé répondant à la formule III
R¹-COX (III)
dans laquelle X représente un halogène ou un groupe éliminable usuel dans les réactions d'acylation.

8. Produit approprié à l'utilisation pour la lutte contre les mycètes nuisibles, contenant une quantité efficace d'un composé de formule I selon les revendications 1 à 6 et au moins un produit auxiliaire de formulation usuel.

9. Procédé pour combattre les mycètes nuisibles, caractérisé par le fait que l'on traite les mycètes nuisibles, leur habitat ou les végétaux, aires, matériaux ou locaux qu'on veut protéger contre les mycètes nuisibles, par une quantité fongicide efficace d'un composé de formule I selon les revendications 1 à 6.

10. Utilisation des composés I selon les revendications 1 à 6 pour la lutte contre les mycètes nuisibles.
